# EUROPEAN PATENT SPECIFICATION

(11) **EP 0 912 201 B1**
(45) Date of publication and mention of the grant of the patent: **12.02.2003**
(21) Application number: 97926332.4
(22) Date of filing: 29.05.1997
(51) Int. Cl.: A61L 15/16, A61F 13/15

(54) **A TEXTILE FIBRE REINFORCED ABSORBENT MATERIAL**
DURCH TEXTILFASERN VERSTÄRKTES ABSORBIERENDES MATERIAL
MATIERE ABSORBANTE RENFORCEE PAR DES FIBRES TEXTILES

(30) Priority: 31.05.1996 SE 9602156
(43) Date of publication of application: 06.05.1999
(73) Proprietor: SCA Hygiene Products AB, 405 03 Göteborg (SE)
(72) Inventor: BUSCHKA, Anette, S-416 79 Göteborg (SE); BLOMSTRÖM, Peter, S-416 74 Göteborg (SE); BILLGREN, Tomas, S-429 34 Kullavik (SE)
(74) Representative: Larsson, Karin
(86) International application number: SE9700928
(87) International publication number: WO97045146

(56) References cited:
- WO-A-90/05808
- WO-A-94/10956
- WO-A-95/22656
- US-A- 3 984 898

## Description

The present invention relates to an absorbent material that has improved mechanical strength and that includes a mat of dry-formed cellulose fibres. The invention also relates to a method of producing such a material and to its use.

The inventive material is intended for use as an absorbent structure in an absorbent article, such as a sanitary napkin, tampon, panty liner, incontinence guard, diaper, bed protector, bandage, saliva absorbent and the like. The material may either be used immediately without defibring the material or subsequent to defibring the material and forming a mat for an absorbent core.

It is known from International Patent Application WO 90/05808 to fabricate a pulp web for later defibration with the aid of a dry forming process, so-called dry-formed reel pulp. A controlled flow of flash-dried paper pulp fibres, which may be a thermomechanical pulp, chemithermomechanical pulp, CTMP, or chemical paper pulp, sulphite or sulphate pulp, having a dry solids content of about 80% is delivered with the aid of an air flow to a forming head mounted above a wire and there formed into a web having a weight per unit area of 300-1500 g/m² and a density of 550-1000 kg/m³. The air is removed by suction through a suction box disposed beneath the wire. The process moisture content shall be 5-30%.

The web is pre-pressed so as to slightly reduce the bulk of the web prior to finally pressing the web to a density of 550-1000 kg/m³. The pressed web is sufficiently strong to enable it to be rolled-up or handled in sheet form for storage and transportation purposes. It can readily be defibred and is intended to be transferred in a fluffed state for use in the manufacture of absorbent bodies in diapers, sanitary napkins and like products.

One of the advantages afforded by this material is that the pulp obtains a low hydrogen bond concentration, and consequently the amount of energy required to defibre the pulp is less than that required to defibre a conventional wet-formed pulp. The lower number of hydrogen bonds also enables the material to be heavily compressed while retaining the low defibring energy input requirement. Superabsorbents may also be admixed with the dry-formed material during its manufacture, which is not possible in the manufacture of a wet-formed material.

It is also known from International Patent Application WO 94/10956 that such a material can be used directly as an absorbent structure, without defibring and fluff-forming the material. In this regard, the absorbent structure includes a dry-formed sheet containing cellulose fibres and having a density of between 0.2 and 1 g/cm³ and a weight per unit area of between 30 and 2000 g/m². This material has very good dispersion properties and swells markedly when wetted. When the material is softened, soft absorbent cores can also be obtained while retaining the good dispersion properties.

One drawback with the aforedescribed dry-formed materials is their mechanical weakness, both in a non-defibred state and in a defibred and mat-formed state.

To overcome this problem WO 94/10956 suggests increasing the network strength by reinforcing the structure of the dry-formed roll pulp by adding reinforcing fibres, binding fibres or binding agent to the cellulose fibre mixture. It should also be possible to incorporate a reinforcing layer of plastic, non-woven, net or threads in the absorbent structure or fastening a reinforcing layer on one or both sides of the material.

When adding reinforcing fibres to flash-dried pulp fibres during dry-laying or to cellulose fibres obtained by defibration of wet-formed roll or sheet pulp it is necessary to use short and rigid reinforcing fibres. Long and soft fibres will tangle together when included in the air flow of cellulose fibres.

When using a reinforcing layer it is necessary to make the cellulose fibre layer and reinforcing layer adhere to each other, for instance by adding an adhesive. However, the adhesive will make the end product more rigid and also limit the absorbancy.

US 3,984,898 describes a method and an apparatus for producing multilayer fibrous mats comprising alternate short and long fibre layers. The apparatus comprises a long fibre defibrating unit feeding long fibres to a chute means directing the fibres to a wire. A short fibre defibrating unit shreds and defibrates woodpulp, disperses the obtained fibres and deposits them on the long fibre mat. The intention is to achieve interfibre bonds at the interface between the layers. However, the mats formed will only have weak bonds easily broken and delamination will easily occur. Therefore, spraying of the very thin long fibre layer or layers with binder is recommended. Further, the long fibre web is deposited by the lickerin method which gives a low productivity. It is only possible to obtain a weight per unit area of about 3 g/m². It may therefore be necessary to use more than one lickerin unit.

The object of the present invention is to provide a dry-formed material that is suited both for defibration and mat-forming and for direct use as an absorbent material, optionally after softening the material.

This object is achieved with an absorbent material that comprises a mat of dry-formed cellulose fibres and is characterized in that the mat is integrated with an air-laid nonwoven gauze layer of reinforcing textile fibres. The nonwoven layer and the cellulose fibre mat may be integrated with one another to an extent in which a homogenous or essentially homogenous material is obtained. The inventive absorbent material may also be a clearly defined multi-layer material in which the boundary layers are integrated with one another. When one layer is thin in relation to the other, the thinner layer may be fully integrated in the thicker layer, which then includes an essentially homogenous distribution of the fibres from the other layer within a zone.

The inventive material may conveniently have a density of 0.1-1 g/cm³, particularly 0.2-0.9 g/cm³, preferably 0.3-0.8 g/cm³ and most preferably 0.4-0.7 g/cm³. The weight per unit area may be 30-2000 g/m², suitably 50-1500 g/m², particularly 100-1000 g/m² and preferably 200-800 g/m².

According to the invention, the material may be produced by air-laying textile fibres on a wire to form a non-woven gauze, whereafter mat-forming of the cellulose fibres is effected on the textile fibre non-woven gauze, e.g. with the aid of an air-laying apparatus. The cellulose fibres will penetrate into the non-woven gauze and be integrated therewith.

To obtain the integration of the short fibre cellulose layer and the longer fibre textile layer this second layer must be a non-woven gauze enabling the cellulose fibres to effectively penetrate the fibre interstices and thus form an integrated layer with the textile fibres.

Formation of the non-woven gauze layer requires the use of a card, for instance a Fehrer K21 card. The simple laying down of long fibres on a wire described in US 3,984,898 will not produce a porous, easily penetrated gauze. Therefore, the layers in the product obtained according to US 3,984,898 requires spraying with a bonding agent to achieve a sufficient degree of interbonding. On the contrary, the present reinforced product is an integrated article not requiring any bonding agent.

Textile fibres are very likely to tangle together. When cellulose fibres and textile fibres are delivered to a wire simultaneously, the latter fibres will not be uniformly distributed unless they are sufficiently short and rigid. The present invention overcomes this problem.

The reinforcing fibres will conveniently have a length of 10-100 mm, particularly 32-60 mm. Fibres of this length cannot be used in the reinforcement of wet-formed pulp.

One advantage of using textile fibre reinforcement in the dry-formed material is that the low hydrogen bond concentration of the material enables it to be defibred in a gentler fashion than wet-formed pulp, without damage to the fibres. The reinforcing effect is thus retained when defibring the material and in the subsequent mat-forming process. The long textile fibres provide a better reinforcement and are also cheaper than the short reinforcing fibres used in wet-formed pulp.

The textile fibres will conveniently have a gauge or linear density of 1-30 dtex, preferably 1-20 dtex. When reinforcing pulp that is to be defibred, the textile fibres will have a preferred gauge of 1-5 dtex.

When the material is intended to be defibred for forming an absorbent core, the material will conveniently contain 1-10%, particularly 2-8%, preferably 3-6% reinforcing fibres calculated on the weight of the material in a dry state.

When the material is intended for direct use, without intermediate defibration, the reinforcing layer may similarly correspond to 1-10%, particularly 2-8%, preferably 3-6% reinforcing fibres, calculated on the weight of the material in a dry state. The layer will then function essentially as a reinforcement. However, this layer can also impart to the two-layer material a soft surface that may also have a suitable colour, particularly may be whiter than the cellulose layer. It is also possible in this case to use a much thicker layer and therewith produce a two-layer material in which the textile fibre layer, for instance, functions as an acquisition layer and the cellulose layer as a storage layer. In this case, the cellulose layer may include superabsorbents and be made relatively thin. The weight ratio between the layers may then be from 20:80 to 80:20, particularly from 35:75 to 75:35.

When the textile fibre layer is to function as an acquisition layer, the textile fibres will preferably be relatively coarse, e.g. have a gauge of 5-30 dtex, particularly 10-25 dtex and preferably 15-20 dtex.

The weight per unit area of the textile fibre layer is suitably at least 5 g/m², especially at least 8 g/m² and preferably about 10-20 g/m², when this layer is intended as a reinforcing layer. When the non-woven gauze layer is to be used as an acquisition layer, the weight per unit area may be higher, for instance 20-1200 g/m², especially 40-800 g/m² and preferably 60-700 g/m².

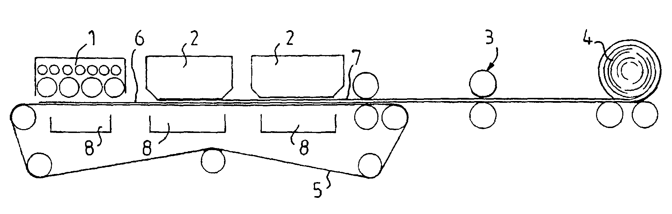

The invention will now be described with reference to the accompanying drawing, which illustrates schematically an inventive method for the manufacture of the inventive absorbent material.

The inventive absorbent material is produced by air-laying textile fibres on a wire 5 to form a non-woven gauze 6 with an air-doffing apparatus 1, e.g. with the aid of a Fehrer K21 card. The non-woven gauze, which becomes anchored to the wire in the air-laying process, then passes at least one cellulose pulp air-laying apparatus, such as a Kröyer head 2 or the like, whereby a mat of flash-dried cellulose fibres is formed on the textile fibre non-woven gauze. Suction boxes 8 are disposed beneath the wire 5 opposite the card 1 and the Kröyer head or Kröyer heads 2. The two-layer material is removed from the wire 5 and passes through a calendar 3, whereafter the material is rolled-up as finished material 4.

The finished material 4 can be used directly as an absorbent structure with no intermediate defibration and optionally after being softened. The dry-laid cellulose material retains its effective absorptive and dispersive properties. The mechanical strength of the material is greatly improved by the textile fibre non-woven gauze. Because the non-woven gauze is orientated towards one surface of the absorbent material, it can also be used to provide coloured material on one side of the absorbent material for instance, or to form a two-layer material whose layers have mutually different properties. This may be achieved by using different types of reinforcing fibres that have different suction properties and different thicknesses for instance. This enables the suction properties of the material to be influenced.

It is also possible to "layer" different absorption properties in a structure, by using a sufficiently large amount of textile fibres that possess other suction characteristics than the pulp and then folding the material double or laying the material in several layers.

Because the dry-formed pulp has low binding energy, the material is easy to defibre and to form a mat reinforced with textile fibres. The textile fibres retain their length and other properties to a substantial extent. This results in an homogenous, reinforced, absorbent structure.

### Example 1

Wet-formed HTCTMP with an admixture of about 3% rayon fibres, 12 mm, 1.7 dtex was produced by way of reference. The material was defibred and mat-formed. A network strength of 5.5 N was obtained. A network strength of 3-4 N was obtained with pure HTCTMP.

Samples according to the invention were then prepared. The results are set forth in the following Table 1.

**Table 1**

| Sample No. | Admixture of 1.7 dtex viscose | | Network strength N |
|---|---|---|---|
| | % | Fibre length | |
| Ref. | about 3 | 12 | 5.5 |
| 1 | 2.3 | 40 | 5.3 |
| 2 | 5.3 | 40 | 4.8 |
| 3 | 3.0 | 50% 12, 50% 40 | 5.0 |
| 4 | 5.3 | " | 6.0 |
| 5 | 5.3 | " | 6.1 |
| Pure CTMP | 0 | | 3-4 |

A network strength that equalled the network strength of the reference material was obtained already with an admixture of 2.3% 40 mm fibres. An admixture of 5.3% of a mixture of 50% 12 mm and 50% 40 mm viscose fibres gave a network strength of about 6%. Samples 4 and 5 were comprised of the same material where defibration treatment was carried out from the cellulose side in the first case and from the viscose fibre side in the second case. Essentially, die same results were obtained.

### Example 2

Pure dry-formed HTCTMP and HTCTMP dry-formed on a polyester non-woven gauze "Grilene" comprised of fibres of 38 mm, 1.7 dtex, were produced. The density, weight per unit area, maximum shear force, elongation at maximum shear force and ultimate elongation were measured, the last-mentioned properties before and after ageing treatment under 50 cycles. In respect of the textile fibre reinforced material, these magnitudes were determined without softening the sample (a) and after softening the sample (b), respectively. Residual shear force was calculated as the ratio between the maximum shear forces subsequent and prior to ageing respectively. As evident from the following Table, this value increased from 33% in the case of pure HTCTMP to 95-125% in respect of the inventive material. Other magnitudes, particularly stretchability, were also greatly improved after fibre reinforcement.

**Table 2**

| Sample | | Pure CTMP | 6 | | 7 | | 8 | | 9 | |
|---|---|---|---|---|---|---|---|---|---|---|
| | | | a | b | a | b | a | b | a | b |
| Fibre content | | 0 | 9.7 | | 6.9 | | 5.6 | | 4.2 | |
| Density before compression, kg/m³ | | 360 | | | | | | | | |
| Density after compression, kg/m³ | | 420 | 467 | 350 | 657 | 356 | 734 | 390 | 776 | 383 |
| Weight per unit area before compression, g/m² | | 440 | | | | | | | | |
| Weight per unit area after compression, g/m² | | 450 | 440 | 421 | 456 | 426 | 459 | 418 | 440 | 435 |
| Max. shear force, N | I | 1.4 | 3.3 | 2.4 | 2.8 | 2.1 | 2.4 | 1.7 | 2.3 | 1.7 |
| | II | 0.5 | 3.5 | 3.0 | 2.9 | 2.1 | 2.3 | 1.5 | 2.2 | 1.6 |
| Elongation at max. | I | 31 | 145 | 116 | 90 | 84 | 52 | 44 | 50 | 41 |
| shear force, % | II | 32 | 158 | 168 | 159 | 158 | 47 | 132 | 80 | 104 |
| Ultimate elongation, % | I | 67 | 294 | 301 | 280 | 267 | 287 | 291 | 275 | 279 |
| | II | 113 | 293 | 319 | 267 | 294 | 284 | 304 | 311 | 279 |
| Residual shear force, % | | 33 | 107 | 125 | 104 | 101 | 99 | 87 | 96 | 95 |
| I = before ageing, II = after ageing. | | | | | | | | | | |

## Claims

1. An absorbent material comprising a mat of dry-laid cellulose fibres, **characterized in that** the mat is integrated with an air-laid non-woven gauze comprised of reinforcing textile fibres.

2. An absorbent material according to Claim 1, **characterized in that** the reinforcing textile fibres have a length of 10-100 mm, particularly 20-80 mm, and then preferably 32-60 mm.

3. An absorbent material according to Claim 1 or 2, **characterized in that** it includes up to 10% reinforcing fibres, calculated on the weight of the material.

4. An absorbent material according to Claim 3, **characterized in that** it contains 2-8%, preferably 3-6% reinforcing fibres.

5. An absorbent material according to any one of Claims 1-4, **characterized in that** the reinforcing fibres are cotton fibres, synthetic fibres or chemical fibres.

6. An absorbent material according to Claim 5, **characterized in that** the reinforcing fibres are rayon fibres or polyester fibres.

7. An absorbent material according to Claim 1, **characterized in that** the weight ratio between the cellulose fibre layer and the textile fibre layer is from 20:80 to 80:20, particularly from 35:75 to 75:35.

8. An absorbent material according to Claim 7, **characterized in that** the textile fibres have a gauge of 5-30 dtex, suitably 10-25 dtex, and particularly 15-20 dtex.

9. An absorbent material according to Claim 3 or 4, **characterized in that** the textile fibres have a gauge of 1-10 dtex, particularly 1-4 dtex.

10. A method of producing an absorbent material that comprises a mat of dry-laid cellulose fibres integrated with an air-laid non-woven gauze comprised of reinforcing textile fibres, **characterized by** air-forming textile fibres with a card on a wire to form a non-woven gauze, whereafter the cellulose fibres are formed into a mat integrated with the textile fibre non-woven gauze.

11. A method according to Claim 10, **characterized in that** the reinforcing textile fibres have a length of 10-100 mm, particularly 20-80 mm, and then preferably 32-60 mm.

12. A method according to Claim 10 or 11, **characterized in that** the material contains up to 10% reinforcing fibres, calculated on the weight of the material.

13. A method according to Claim 12, **characterized in that** the material contains 3-8% reinforcing fibres.

14. A method according to any one of Claims 10-13, **characterized in that** the reinforcing fibres are cotton fibres, synthetic fibres or chemical fibres.

15. A method according to Claim 14, **characterized in that** the reinforcing fibres are rayon fibres or polyester fibres.

16. A method according to Claim 10 or 11, **characterized in that** the weight ratio between the cellulose fibre layer and the textile fibre layer is from 20:80 to 80:20, particularly from 35:75 to 75:35.

17. The use of an absorbent material that comprises a mat of dry-laid cellulose fibres integrated with an air-laid non-woven gauze of reinforcing textile fibres as an absorbent structure in an absorbent product, such as a diaper, sanitary napkin, mattress protector, for instance.

18. The use according to Claim 17, **characterized in that** the absorbent material is used directly as an absorbent structure without intermediate defibration.

19. The use according to Claim 17, **characterized in that** the absorbent material is defibred and mat-formed into an absorbent core that is used as an absorbent structure.

20. An absorbent structure comprising cellulose fibres reinforced with textile fibres, **characterized in that** the structure is produced by defibrating and mat-forming an absorbent material comprising a dry-laid mat of cellulose fibres integrated with an air-laid non-woven gauze of long reinforcing textile fibres.

21. A method of producing an absorbent structure comprising cellulose fibres and reinforcing textile fibres, **characterized in that** a mat of dry-formed cellulose fibres integrated with an air-laid non-woven gauze of reinforcing textile fibres is defibrated and mat-formed.

## Patentansprüche

1. Absorptionsmaterial mit einer Matte aus trockengelegten Zellulosefasern, **dadurch gekennzeichnet, dass** die Matte mit einer luftgelegten nicht gewebten Gaze integriert ist, die aus verstärkenden Textilfasern besteht.

2. Absorptionsmaterial nach Anspruch 1, **dadurch gekennzeichnet, dass** die verstärkenden Textilfasern eine Länge von 10 bis 100 mm, insbesondere 20 bis 80 mm, und dann vorzugsweise 32 bis 60 mm aufweisen.

3. Absorptionsmaterial nach Anspruch 1 oder 2, **dadurch gekennzeichnet, dass** es bis 10% verstärkende Fasern, berechnet anhand des Gewichts des Materials, aufweist.

4. Absorptionsmaterial nach Anspruch 3, **dadurch gekennzeichnet, dass** es 2 bis 8%, insbesondere 3 bis 6% verstärkende Fasern enthält.

5. Absorptionsmaterial nach einem der Ansprüche 1 bis 4, **dadurch gekennzeichnet, dass** die verstärkenden Fasern Baumwollfasern, synthetische oder chemische Fasern sind.

6. Absorptionsmaterial nach Anspruch 5, **dadurch gekennzeichnet, dass** die verstärkenden Fasern Rayonfasern oder Polyesterfasern sind.

7. Absorptionsmaterial nach Anspruch 1, **dadurch gekennzeichnet, dass** das Gewichtsverhältnis zwischen der Zellulosefaserschicht und der Textilfaserschicht von 20:80 bis 80:20, insbesondere von 35:75 bis 75:35 ist.

8. Absorptionsmaterial nach Anspruch 7, **dadurch gekennzeichnet, dass** die Textilfasern eine Feinheit von 5 bis 30 dtex, in geeigneter Weise 10 bis 25 dtex, und insbesondere 15 bis 20 dtex aufweist.

9. Absorptionsmaterial nach Anspruch 3 oder 4, **dadurch gekennzeichnet, dass** die Textilfasern eine Feinheit von 1 bis 10 dtex, insbesondere 1 bis 4 dtex aufweisen.

10. Verfahren zur Herstellung eines Absorptionsmaterials mit einer Matte aus trockengelegten Zellulosefasern, die mit einer luftgelegten nicht gewebten Gaze, die aus verstärkenden Textilfasern besteht, integriert ist, **gekennzeichnet durch** Luftformen von Textilfasern mit einer Karde auf einem Sieb, um eine nicht gewebte Gaze auszubilden, woraufhin die Zellulosefasern in eine Matte geformt werden, die mit der nicht gewebten Textilfaser-Gaze integriert ist.

11. Verfahren nach Anspruch 10, **dadurch gekennzeichnet, dass** die verstärkenden Textilfasern eine Länge von 10 bis 100 mm, insbesondere 20 bis 80 mm und dann vorzugsweise 32 bis 60 mm aufweisen.

12. Verfahren nach Anspruch 10 oder 11, **dadurch gekennzeichnet, dass** das Material bis zu 10% verstärkende Fasern, berechnet anhand des Gewichts des Materials, aufweist.

13. Verfahren nach Anspruch 12, **dadurch gekennzeichnet, dass** das Material 3 bis 8% verstärkende Fasern enthält.

14. Verfahren nach einem der Ansprüche 10 bis 13, **dadurch gekennzeichnet, dass** die verstärkenden Fasern Baumwollfasern, synthetische oder chemische Fasern sind.

15. Verfahren nach Anspruch 14, **dadurch gekennzeichnet, dass** die verstärkenden Fasern Rayonfasern oder Polyesterfasern sind.

16. Verfahren nach Anspruch 10 oder 11, **dadurch gekennzeichnet, dass** das Gewichtsverhältnis zwischen der Zellulosefaserschicht und der Textilfaserschicht von 20:80 bis 80:20, insbesondere von 35:75 bis 75:35 beträgt.

17. Verwendung eines Absorptionsmaterials mit einer Matte aus trockengelegten Zellulosefasern, die mit einer luftgelegten nicht gewebten Gaze aus verstärkenden Textilfasern integriert sind, als eine Absorptionsstruktur in einem Absorptionsprodukt wie z.B. einer Windel, einer Hygienebinde, einem Matratzenschutz.

18. Verwendung nach Anspruch 17, **dadurch gekennzeichnet, dass** das Absorptionsmaterial unmittelbar als eine Absorptionsstruktur ohne Zwischenentfaserung verwendet wird.

19. Verwendung nach Anspruch 17, **dadurch gekennzeichnet, dass** das Absorptionsmaterial entfasert wird und in einen Absorptionskern mattengeformt wird, der als eine Absorptionsstruktur verwendet wird.

20. Absorptionsstruktur mit Zellulosefasern, die mit Textilfasern verstärkt sind, **dadurch gekennzeichnet, dass** die Struktur durch Entfasern und Mattenformen eines Absorptionsmaterials hergestellt wird, das eine trockengelegte Matte aus Zellulosefasern aufweist, die mit einer luftgelegten nicht gewebten Gaze aus langen verstärkenden Textilfasern integriert ist.

21. Verfahren zur Herstellung einer Absorptionsstruktur mit Zellulosefasern und verstärkenden Textilfasern, **dadurch gekennzeichnet, dass** eine Matte aus trockengeformten Zellulosefasern, die mit einer luftgelegten nicht gewebten Gaze aus verstärkenden Textilfasern integriert ist, entfasert wird, und mattengeformt wird.

## Revendications

1. Matériau absorbant comprenant un mat de fibres de cellulose déposées à sec, **caractérisé en ce que** le mat est intégré avec une gaze non tissée déposée au moyen d'air, constituée de fibres textiles de renfort.

2. Matériau absorbant selon la revendication 1, **caractérisé en ce que** les fibres textiles de renfort ont une longueur de 10 à 100 mm, en particulier de 20 à 80 mm, et plus préférablement de 32 à 60 mm.

3. Matériau absorbant selon la revendication 1 ou 2, **caractérisé en ce qu**'il comprend jusqu'à 10 % de fibres de renfort, pourcentage calculé sur le poids du matériau.

4. Matériau absorbant selon la revendication 3, **caractérisé en ce qu**'il contient de 2 à 8 %, de préférence de 3 à 6 % de fibres de renfort.

5. Matériau absorbant selon l'une quelconque des revendications 1 à 4, **caractérisé en ce que** les fibres de renfort sont des fibres de coton, des fibres synthétiques ou des fibres chimiques.

6. Matériau absorbant selon la revendication 5, **caractérisé en ce que** les fibres de renfort sont des fibres de rayonne ou des fibres de polyester.

7. Matériau absorbant selon la revendication 1, **caractérisé en ce que** le rapport de poids entre la couche de fibres de cellulose et la couche de fibres textiles est compris entre 20/80 et 80/20, en particulier entre 35/75 et 75/35.

8. Matériau absorbant selon la revendication 7, **caractérisé en ce que** les fibres textiles ont un titre de 5 à 30 dtex, de façon appropriée de 10 à 25 dtex, et en particulier de 15 à 20 dtex.

9. Matériau absorbant selon la revendication 3 ou 4, **caractérisé en ce que** les fibres textiles ont un titre de 1 à 10 dtex, en particulier de 1 à 4 dtex.

10. Procédé de fabrication d'un matériau absorbant qui comprend un mat de fibres de cellulose déposées à sec intégré avec une gaze non tissée déposée au moyen d'air, constituée de fibres textiles de renfort, **caractérisé par** le traitement à l'air de fibres textiles, à l'aide d'une carde sur un tamis toile métallique pour former une gaze non tissée, après quoi les fibres de cellulose sont transformées en un mat intégré avec la gaze non tissée de fibres textiles.

11. Procédé selon la revendication 10, **caractérisé en ce que** les fibres textiles de renfort ont une longueur de 10 à 100 mm, en particulier de 20 à 80 mm, et plus préférablement de 32 à 60 mm.

12. Procédé selon la revendication 10 ou 11, **caractérisé en ce que** le matériau contient jusqu'à 10 % de fibres de renfort, pourcentage calculé sur le poids du matériau.

13. Procédé selon la revendication 12, **caractérisé en ce que** le matériau contient de 3 à 8 % de fibres de renfort.

14. Procédé selon l'une quelconque des revendications 10 à 13, **caractérisé en ce que** les fibres de renfort sont des fibres de coton, des fibres synthétiques ou des fibres chimiques.

15. Procédé selon la revendication 14, **caractérisé en ce que** les fibres de renfort sont des fibres de rayonne ou des fibres de polyester.

16. Procédé selon la revendication 10 ou 11, **caractérisé en ce que** le rapport de poids entre la couche de fibres de cellulose et la couche de fibres textiles est compris entre 20/80 et 80/20, en particulier entre 35/75 et 75/35.

17. Utilisation d'un matériau absorbant qui comprend un mat de fibres de cellulose déposées à sec intégré avec une gaze non tissée déposée au moyen d'air constituée de fibres textiles de renfort, en tant que structure absorbante dans un produit absorbant tel qu'une couche-culotte, une serviette hygiénique, un protection de matelas, par exemple.

18. Utilisation selon la revendication 17, **caractérisée en ce que** le matériau absorbant est utilisé directement en tant que structure absorbante sans défibrage intermédiaire.

19. Utilisation selon la revendication 17, **caractérisée en ce que** le matériau absorbant est défibré et mis en mat en formant un noyau absorbant qui est utilisé en tant que structure absorbante.

20. Structure absorbante comprenant des fibres de cellulose renforcées avec des fibres textiles, **caractérisée en ce que** la structure est réalisée en défibrant et en mettant en mat un matériau absorbant comprenant un mat déposé à sec de fibres de cellulose intégré avec une gaze non tissée déposée au moyen d'air de fibres textiles de renfort longues.

21. Procédé de fabrication d'une structure absorbante comprenant des fibres de cellulose et des fibres textiles de renfort, **caractérisé en ce qu'**un mat de fibres de cellulose traitées à sec intégré avec une gaze non tissée de fibres de renfort déposée au moyen d'air est défibrée et mise en mat.
